# EUROPEAN PATENT SPECIFICATION

(11) **EP 3 944 870 B1**
(45) Date of publication and mention of the grant of the patent: **13.03.2024**
(21) Application number: 21188481.2
(22) Date of filing: 29.07.2021
(51) Int. Cl.: A61B 5/00, A61M 5/14, A61M 5/142, A61B 5/145

(54) **INDWELLING DEVICE**
VERWEILVORRICHTUNG
DISPOSITIF À DEMEURE

(30) Priority: 30.07.2020 JP 2020129412
(43) Date of publication of application: 02.02.2022
(73) Proprietor: TERUMO Kabushiki Kaisha, Tokyo 151-0072 (JP)
(72) Inventor: OHASHI, Hirotaka, Tokyo, 163-1450 (JP); SAWAZAKI, Ryoichi, Showa-cho, 409-3853 (JP)
(74) Representative: Casalonga

(56) References cited:
- EP-A1- 3 437 676
- GB-A- 2 423 267
- JP-A- 2013 063 105
- US-A1- 2009 012 472

## Description

### BACKGROUND

### Technical Field

The present invention relates to an indwelling device that is indwelled on a body surface of a living body.

In particular, the present invention relates to an indwelling device such as it may e. g. be found in US 2009/012472 A1.

### Related Art

JP 2013-63105 A discloses a drug administration device (indwelling device) that is indwelled on a body surface in order to administer a drug solution into a body of a living body. The indwelling device includes a main body that supplies a drug solution, and an attaching sheet that is attached to a bottom surface of the main body and is attached to a body surface to indwell the main body.

### SUMMARY

In order to enable this type of indwelling device to be attached to a body surface of various patients, a main body of the indwelling device has a flat bottom surface. However, when the main body is attached to a curved portion of a body surface, followability to the body surface is deteriorated. Therefore, the main body may be peeled off from the attaching member (or the attaching member may be peeled off from the body surface) in a case where the main body is indwelled for a long period of time or the like.

In order to cope with such a problem, in the indwelling device of JP 2013-63105 A, a case of the main body has a plastic region, and the case itself can be deformed in accordance with the curved shape of the body surface. However, in a case of a structure in which the case itself is deformed as described above, there are other problems such as complication of the device and an increase in manufacturing cost.

The present invention relates to the technique of the above-described indwelling device, and an object thereof is to provide an indwelling device that can be stably indwelled on a body surface of a living body with a simple configuration.

In order to achieve the above object, the present invention provides an indwelling device according to independent claim 1. The dependent claims relate to advantageous embodiments.

The above-described indwelling device can be stably indwelled on a body surface of a living body with a simple configuration.

### BRIEF DESCRIPTION OF DRAWINGS

FIG. 1 is a perspective view showing an overall configuration of an indwelling device (drug administration device) according to an embodiment of the present invention;
FIG. 2 is an exploded perspective view showing an indwelling structure of a main body;
FIG. 3 is a plan view showing a state in which a main-body-side attaching sheet, an intermediate member, a lower adhesive body, and an upper adhesive body are stacked;
FIG. 4A is a side cross-sectional view showing an indwelled state of the main body on a body surface;
FIG. 4B is a partial perspective view showing positions of the main body and the main-body-side attaching sheet in the indwelled state;
FIG. 4C is a partial perspective view showing operation when an external force is applied to the main body;
FIG. 5 is an exploded perspective view showing an indwelling structure according to a first modification;
FIG. 6 is an exploded perspective view showing an indwelling structure according to a second modification;
FIG. 7 is an exploded perspective view showing an indwelling structure according to a third modification;
FIG. 8A is a plan view showing an indwelling structure according to a fourth modification;
FIG. 8B is a plan view showing an indwelling structure according to a fifth modification;
FIG. 8C is a plan view showing an indwelling structure according to a sixth modification; and
FIG. 8D is a plan view showing an indwelling structure according to a seventh modification.

### DETAILED DESCRIPTION

Hereinafter, preferred embodiments of the present invention will be described in detail with reference to the accompanying drawings.

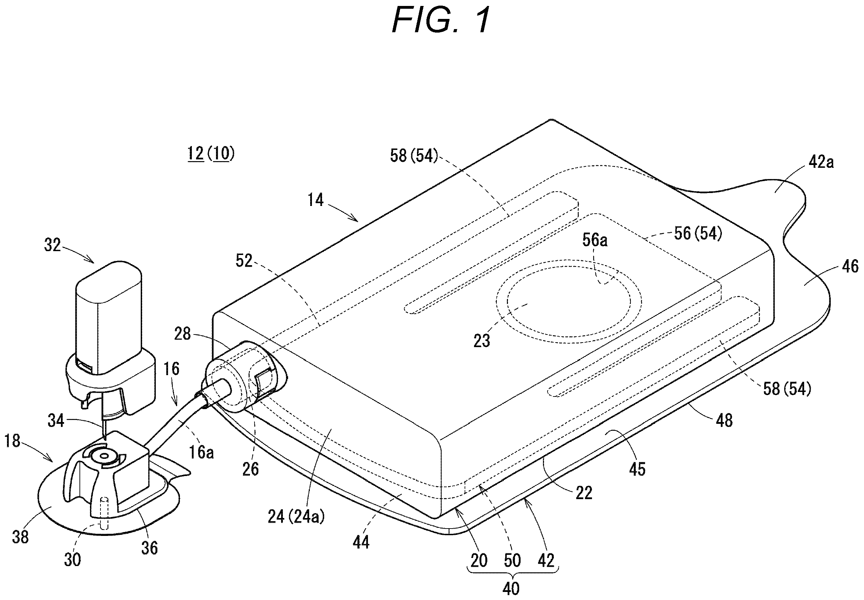

As an indwelling device 10 according to an embodiment of the present invention, a drug administration device 12 shown in FIG. 1 will be representatively described. The drug administration device 12 is a medical device that is indwelled on a body surface of a patient by a medical worker or the like and automatically administers a drug into the body in an indwelled state. Note that the indwelling device 10 according to the present invention is not limited to the drug administration device 12, and can be applied to various devices indwelled on a body surface of a living body. Examples of the device include a component measurement device such as a blood glucose meter, a biosensor or another medical device, and a terminal other than medical devices such as an information processing device and a communication device.

The drug administration device 12 includes a main body 14 that is a drug supply source, a tube 16 connected to the main body 14, and an administration unit 18 connected to the tube 16 and configured to administer a drug solution supplied from the tube 16 to a patient. That is, in the drug administration device 12, the main body 14 and the administration unit 18 are connected to each other via the tube 16, thereby ensuring a certain degree of mutual freedom. Therefore, the drug administration device 12 can easily and accurately position the main body 14 and the administration unit 18 at desired positions on the body surface when indwelled in a living body.

The main body 14 of the drug administration device 12 includes a container for storing a drug, a mechanism unit that delivers the drug from the container to the tube 16 at an appropriate timing, a control unit that controls operation of the mechanism unit, a power source of the mechanism unit and the control unit (both not shown), and the like. Examples of the drug administered by the drug administration device 12 include liquid drugs such as antibody drugs, anticancer agents, chemotherapeutic agents, anesthetics, antibiotics, insulin, blood products, and nutrients. The main body 14 has a case 20 that accommodates these components.

The case 20 has a large rectangular shape in plan view, and is configured as a substantially rectangular housing having a low rectangular shape in a thickness direction. The size of the case 20 is set to, for example, a range in which a length in a longitudinal direction is about 30 mm to 150 mm, a range in which a length in a lateral direction is about 10 mm to 100 mm, and a range in which the thickness is about 5 mm to 20 mm.

In the case 20, a bottom surface 22 which is an indwelling side (a fixing target place) of the body surface is formed in a flat shape. The bottom surface 22 may be formed in a slightly curved surface according to the shape of the body surface to be indwelled. The four side portions of the bottom surface 22 are continuous in an R shape with respect to the four side surface portions 24 of the case 20, and corner portions where the side portions intersect each other are also formed in an R shape. The case 20 (main body 14) constitutes a part of the indwelling structure 40, and is indwelled on the body surface.

The bottom surface 22 is provided with an operation unit 23 for operating the main body 14. The operation unit 23 according to the present embodiment is configured as a round push button. The main body 14 is configured such that the power source is turned off in the product providing state, and the control unit continues the operation of the main body 14 after being turned on once by the pressing of the operation unit 23 by the user. The operation unit 23 is not limited to the one that performs the on/off operation of the power supply, and can be applied to various configurations for operating the indwelling device 10. The main body 14 may be provided with the operation unit 23 at a location other than the bottom surface 22 of the case 20, and may be configured not to be provided with the operation unit 23 when operated by external communication or the like.

The case 20 is preferably made of a hard resin material so as not to be elastically deformable. The resin material of the case 20 is not particularly limited, but for example, a thermoplastic resin such as polyacetal, polyamide, polyester, polycarbonate, polysulfone, polytetrafluoroethylene, polyphenylene sulfide, or polypropylene may be applied. The shape of the case 20 is not limited to a substantially rectangular shape, and various shapes such as a cubic shape, a hemispherical shape, and other three-dimensional shapes can be adopted.

A main-body-side connector 26 for connecting to the tube 16 is provided at one end in the longitudinal direction (direction of arrow A) of the case 20. The main-body-side connector 26 protrudes short from a side surface portion 24a on one end side of the case 20. A mounting structure for mounting the tube 16 is formed on an outer circumferential surface of the main-body-side connector 26.

The tube 16 of the drug administration device 12 is a flexible tube having a flow passage 16a therein which extends by a predetermined length and through which the drug can flow. The length of the tube 16 is not particularly limited, but is set in a range of about 10 mm to 100 mm, for example. A tube side connector 28 attachable to and detachable from the main-body-side connector 26 is fixed to one end portion of the tube 16. The administration unit 18 is fixed to the other end of the tube 16.

The tube side connector 28 includes a hollow needle (not shown). The hollow needle allows a storage space of the container and the flow passage 16a of the tube 16 to communicate with each other in a state where the tube side connector 28 is mounted to the main-body-side connector 26.

The administration unit 18 of the drug administration device 12 administers a drug to a patient via a catheter 30 inserted into the body (subcutaneously) from the body surface. Before the catheter 30 is indwelled in the patient, the administration unit 18 is assembled to an insertion device 32, and an inner needle 34 of the insertion device 32 penetrates the catheter 30 (FIG. 1 shows a separated state of the administration unit 18 and the insertion device 32 for ease of understanding of the present invention). A user punctures the body with a multiple needle in which the catheter 30 and the inner needle 34 overlap, and withdraws the inner needle 34 from the catheter 30 in the punctured state (detaches the insertion device 32 from the administration unit 18), thereby indwelling the catheter 30 in the patient.

Specifically, the administration unit 18 includes a catheter 30, a hub 36 that holds a proximal end of the catheter 30 and to which the tube 16 is coupled, and an administration unit side attaching sheet 38 that attaches the hub 36 onto the body surface.

Next, the indwelling structure 40 in which the main body 14 of the drug administration device 12 (the indwelling device 10) is indwelled on the body surface will be described with reference to FIGS. 1 to 3. The indwelling structure 40 includes the case 20 of the main body 14, a main-body-side attaching sheet 42 (attaching member) for attaching the main body 14 to the body surface of the living body, and a plate-like intermediate member 50 stacked between the main body 14 and the main-body-side attaching sheet 42.

The main-body-side attaching sheet 42 has a substantially rectangular shape in plan view corresponding to the shape of the bottom surface 22 of the case 20, and has flexibility capable of following the body surface. The main-body-side attaching sheet 42 is formed to be longer than the bottom surface 22 in the longitudinal direction and the lateral direction. Both sides in the longitudinal direction of the main-body-side attaching sheet 42 are formed in an arc shape in which the central portion in the lateral direction is slightly expanded. Both sides in the lateral direction of the main-body-side attaching sheet 42 are connected to both sides in the longitudinal direction with rounded corners interposed therebetween, and extend in parallel and linearly with each other.

A pair of corners located at one end in the longitudinal direction of the main-body-side attaching sheet 42 is formed into a round corner having a small radius of curvature. A pair of corners located at the other end in the longitudinal direction of the main-body-side attaching sheet 42 is formed into a round corner having a large radius of curvature. A short protrusion 42a is continuously provided at the central portion in the lateral direction of the other end in the longitudinal direction of the main-body-side attaching sheet 42. An attaching member side exposure hole 42b that exposes the operation unit 23 of the case 20 is provided slightly closer to the longitudinal direction end than the central portion in the longitudinal direction of the main-body-side attaching sheet 42.

The upper surface of the main-body-side attaching sheet 42 has a fixing region 44 of the main body 14 in a range from one end in the longitudinal direction to beyond the central portion in the longitudinal direction. The fixing region 44 coincides with the fixing range of the intermediate member 50. The main-body-side attaching sheet 42 has a margin region 45 for exposing the upper surface of the main-body-side attaching sheet 42 around the fixing region 44 in the fixed state of the main body 14. The margin region 45 is formed to be narrow on one end side in the longitudinal direction and both end sides in the lateral direction, and formed to be wide on the other end side in the longitudinal direction.

The main-body-side attaching sheet 42 is formed by stacking a base material 46 and an adhesive layer 48. An appropriate material is preferably applied to the base material 46 in consideration of stretchability, followability, air permeability, and the like. Examples of the material of the base material 46 include polyethylene, polyester, polyurethane, nylon, polyolefin, cotton fabric, woven fabric, and nonwoven fabric. The base material 46 may be configured to have functions such as water repellency, hydrophilicity, irregularities, air holes, moisture permeability, and flexibility by arbitrary processing.

The adhesive layer 48 is formed on the entire lower surface (one surface) of the main-body-side attaching sheet 42 facing the body surface. As a material of the adhesive layer 48, for example, an acrylic adhesive, a silicone-based adhesive, a urethane-based adhesive, a synthetic rubber-based adhesive, or the like is applied. The lower surface of the main-body-side attaching sheet 42 may have not only the adhesive layer 48 provided on the entire surface but also a non-adhesive region where the adhesive layer 48 is not applied to a part of the lower surface (for example, the protrusion 42a).

The intermediate member 50 is a flat plate-shaped member that is attached to the bottom surface 22 of the main body 14 and attached to the main-body-side attaching sheet 42. Therefore, the indwelling structure 40 includes a lower adhesive body 60 (first fixing portion) that fixes the main-body-side attaching sheet 42 and the intermediate member 50, and the upper adhesive body 70 (second fixing portion) that fixes the case 20 of the main body 14 and the intermediate member 50. The lower adhesive body 60 is provided on the lower surface (one surface) of the intermediate member 50. The upper adhesive body 70 is provided on the upper surface (the other surface) of the intermediate member 50.

The intermediate member 50 is formed sufficiently thinner than the thickness of the case 20, and can be elastically deformed (curved) such that the plate surface is warped. The thickness of the intermediate member 50 is not particularly limited, but may be set in a range of, for example, about 0.1 mm to 3 mm. For example, the elastic modulus of the intermediate member 50 is preferably set to be larger than the elastic modulus of the body surface (or to the same extent or to a slightly smaller extent).

The intermediate member 50 has a substantially rectangular shape corresponding to the shape of the case 20 (bottom surface 22) in plan view. The planar shape of the intermediate member 50 is formed to match (or resemble to be slightly smaller than) the bottom surface 22 of the main body 14. The intermediate member 50 includes a base 52 constituting a substantially half of one end side in the longitudinal direction of the intermediate member 50 and a plurality of elastic pieces 54 constituting a substantially half of the other end side in the longitudinal direction of the intermediate member 50. In the longitudinal direction of the intermediate member 50, the ratio of the length of each of the base 52 and the plurality of elastic pieces 54 to the entire length of the intermediate member 50 is not particularly limited. In the longitudinal direction of the intermediate member 50, for example, the length of the base 52 may be set in a range of 20% to 80% of the length of the intermediate member 50, and the lengths of the plurality of elastic pieces 54 may be set according to a ratio of the length of the base 52 to the length of the intermediate member 50.

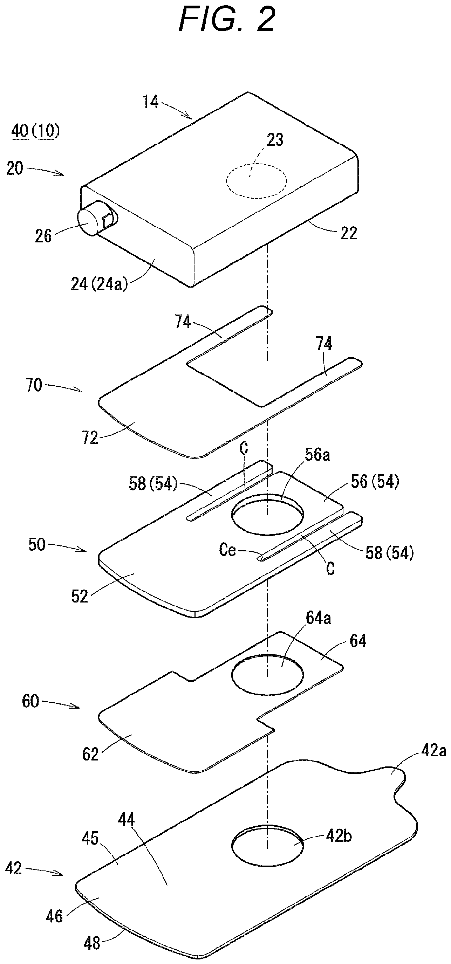

The base 52 is attached to substantially a half of one end side in the longitudinal direction of the case 20 (bottom surface 22) and attached to substantially a half of one end side in the longitudinal direction of the main-body-side attaching sheet 42. Therefore, as shown in FIG. 2, the lower surface of the base 52 is attached to the lower adhesive body 60, and the upper surface of the base 52 is attached to the upper adhesive body 70. A pair of corners on one end side in the longitudinal direction of the base 52 is formed into a round corner matching the shape of the bottom surface 22. The base 52 is disposed at a position in the vicinity of the main-body-side connector 26 (one end side of the main body 14), so that it is possible to enhance the attachment of the outflow portion of the drug of the main body 14 via the main-body-side attaching sheet 42.

The plurality of elastic pieces 54 include a central piece 56 provided at the center in the lateral direction of the intermediate member 50 and a pair of arm pieces 58 provided on both sides in the lateral direction of the central piece 56. Each of the central piece 56 and the pair of arm pieces 58 is flush with the base 52. A clearance C is provided between the central piece 56 and the pair of arm pieces 58 to separate them from each other.

The central piece 56 is formed in a rectangular shape extending from the other end side of the base 52 toward the other end side in the longitudinal direction of the intermediate member 50 by a predetermined length. For example, the width of the central piece 56 in the lateral direction is set to be wide in a range of about 2 to 10 times the width of one arm piece 58.

In the indwelling structure 40, the central piece 56 constitutes a first extending portion that is attached to the main-body-side attaching sheet 42 but is not attached to the case 20 (bottom surface 22). Therefore, the lower adhesive body 60 is attached to the lower surface of the central piece 56, while the upper adhesive body 70 is not attached to the upper surface of the central piece 56.

The plate surface of the central piece 56 is provided with an intermediate member side exposure hole 56a that is formed to have a larger diameter than the attaching member side exposure hole 42b and exposes the operation unit 23. The intermediate member 50 is attached to the main-body-side attaching sheet 42 such that the attaching member side exposure hole 42b is accommodated inside the intermediate member side exposure hole 56a.

The pair of arm pieces 58 is formed in a long plate shape extending from the other end side of the base 52 toward the other end side in the longitudinal direction of the intermediate member 50 by the same length as the central piece 56. Note that the extension length of the pair of arm pieces 58 may be different from the extension length of the central piece 56. In the pair of arm pieces 58, the outer side in the width direction is linearly continuous with one lateral side and the other lateral side of the base 52.

The pair of clearances C provided between the central piece 56 and the pair of arm pieces 58 is set to a width smaller than the width of the pair of arm pieces 58, for example, and extends along the longitudinal direction of the intermediate member 50. An arc-shaped edge portion Ce in contact with the base 52 is formed at one end of the clearance C (a root portion where the central piece 56 and the pair of arm pieces 58 are coupled to the base 52).

The pair of arm pieces 58 is not attached to the main-body-side attaching sheet 42, but constitutes a second extending portion of the indwelling structure 40 attached to the case 20 (bottom surface 22). Therefore, the lower adhesive body 60 is not attached to the lower surface of the arm piece 58, while the upper adhesive body 70 is attached to the upper surface of the arm piece 58.

Examples of the constituent material of the intermediate member 50 include polymer materials such as polyethylene, polypropylene, polyethylene terephthalate, polyvinyl chloride, polystyrene, ABS resin, acrylic, polyamide, polyester, and fluororesin, or mixtures thereof.

The lower adhesive body 60 is a double-sided tape having adhesive layers on a lower surface and an upper surface thereof. The upper adhesive body 70 is a double-sided tape having adhesive layers on a lower surface and an upper surface thereof. The lower adhesive body 60 and the upper adhesive body 70 are formed in a sheet shape thinner than the thickness of the intermediate member 50, and have flexibility. The lower adhesive body 60 and the upper adhesive body 70 may be configured by directly applying an adhesive (for example, the materials mentioned for the adhesive layer 48 of the main-body-side attaching sheet 42) to the lower surface and the upper surface of the intermediate member 50.

The lower adhesive body 60 has a lower base attaching region 62 attached to the entire lower surface of the base 52 and a lower elastic piece attaching region 64 attached to the entire lower surface of the central piece 56. The lower base attaching region 62 and the lower elastic piece attaching region 64 are continuous with the same thickness. The lower elastic piece attaching region 64 has an adhesive-member-side exposure hole 64a at a position corresponding to the intermediate member side exposure hole 56a. The lower base attaching region 62 only needs to firmly fix the main-body-side attaching sheet 42 and the base 52, and may be partially provided on the lower surface of the base 52. Similarly, the lower elastic piece attaching region 64 may also be partially provided on the lower surface of the central piece 56.

The upper adhesive body 70 has an upper base attaching region 72 attached to the base 52 and a pair of upper elastic piece attaching regions 74 attached to the pair of arm pieces 58. The upper base attaching region 72 and the pair of upper elastic piece attaching regions 74 are continuous with each other with the same thickness. The upper base attaching region 72 only needs to firmly fix the main body 14 and the base 52, and may be partially provided on the upper surface of the base 52. Similarly, the pair of upper elastic piece attaching regions 74 may also be partially provided on the upper surfaces of the pair of arm pieces 58.

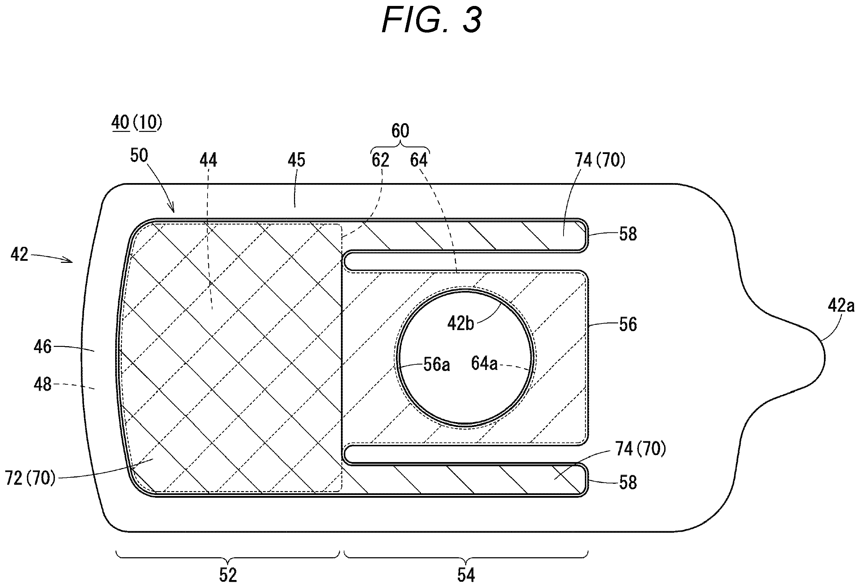

Next, a state where the main-body-side attaching sheet 42, the intermediate member 50, the lower adhesive body 60, and the upper adhesive body 70 are attached will be described with reference to FIG. 3. The intermediate member 50 is attached to the main-body-side attaching sheet 42 by the lower adhesive body 60. However, as described above, in the intermediate member 50, the base 52 and the central piece 56 are attached to the main-body-side attaching sheet 42, but the pair of arm pieces 58 is not attached to the main-body-side attaching sheet 42. The intermediate member 50 (the base 52 and the central piece 56) harder than the main-body-side attaching sheet 42 can hold a wide range of the plane of the main-body-side attaching sheet 42 and suppress wrinkles and slack of the main-body-side attaching sheet 42.

In the intermediate member 50, the base 52 and the pair of arm pieces 58 are attached to the case 20, but the central piece 56 is not attached to the case 20. In this case, in the intermediate member 50, the pair of arm pieces 58 is attached to the vicinities of both sides in the lateral direction of the case 20, so that the attaching state of the case 20 and the intermediate member 50 can be stabilized, and displacement of the case 20 can be suppressed.

The drug administration device 12 (the indwelling device 10) according to the present embodiment is basically configured as described above, and its operation will be described below.

The drug administration device 12 is provided to the user in a state where, while the administration unit 18 and the insertion device 32 are assembled, the tube 16 coupled to the administration unit 18 and the main body 14 are separated. For example, at the time of indwelling the drug administration device 12, the user sequentially performs an administration unit indwelling step of indwelling the administration unit 18, a connecting step of connecting the tube 16 coupled to the administration unit 18 and the main body 14, and a main body indwelling step of indwelling the main body 14.

By the above-described indwelling work, the main body 14 of the drug administration device 12 is fixed on the body surface via the indwelling structure 40. At the time of indwelling, the main body 14 is activated based on ON operation of the operation unit 23. At this time, since the operation unit 23 is exposed through the attaching member side exposure hole 42b and the intermediate member side exposure hole 56a, the user can easily operate the operation unit 23. Even if the operation of the operation unit 23 is forgotten and the main-body-side attaching sheet 42 is attached to the body surface, the operation unit 23 can be exposed by curving the central piece 56 without peeling the main-body-side attaching sheet 42 from the body surface.

After being indwelled, the drug administration device 12 automatically administers the drug to the patient under the control of the control unit. The indwelling period (administration period of the drug) of the drug administration device 12 is set to, for example, a range of about one week to four weeks.

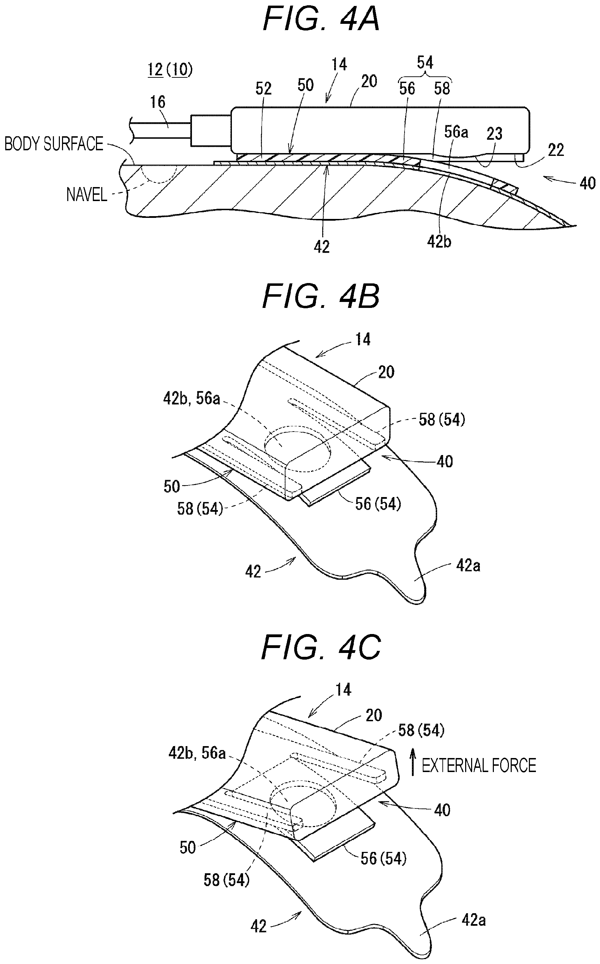

For example, as shown in FIGS. 4A and 4B, in the indwelling state of the main body 14, the main-body-side attaching sheet 42 is attached to a curved portion of the abdomen (body surface) of the patient. At this time, the base 52 of the intermediate member 50 fixed to both the main body 14 and the main-body-side attaching sheet 42 faces the body surface in a narrow range of about half of the entire length of the intermediate member 50 in the longitudinal direction. The entire one end side of the main-body-side attaching sheet 42 including the fixing region 44 of the base 52 and the margin region 45 on the one end side is in close contact with the body surface. In particular, the drug administration device 12 is indwelled so that one end side of the main body 14 faces the center (navel) side of the abdomen at a substantially flat portion of the abdomen, and thereby the case 20 can be stably fixed. One end side of the main body 14 is firmly fixed to the main-body-side attaching sheet 42 via the base 52 of the intermediate member 50. Therefore, for example, the intermediate member 50 can release a load applied from the tube 16 to the main-body-side attaching sheet 42 from one end side to the other end side.

In the indwelling state, the central piece 56 on the other end side of the intermediate member 50 can be separated from the main body 14, and is disposed close to the body surface (the main-body-side attaching sheet 42) following the curve of the body surface. Conversely, the pair of arm pieces 58 of the intermediate member 50 can be separated (floated) from the main-body-side attaching sheet 42 while maintaining the fixed state with the bottom surface 22 of the case 20 without following the body surface.

That is, in the indwelling structure 40, while both the case 20 and the body surface are fixed at the base 52, the central piece 56 follows only the body surface (the main-body-side attaching sheet 42), and the pair of arm pieces 58 follows only the case 20. As a result, the other end side (the plurality of elastic pieces 54 side) of the intermediate member 50 is indwelled so as to be able to approach and separate from the body surface.

In the indwelling structure 40 indwelled as described above, even if an external force that peels the main body 14 from the body surface (the main-body-side attaching sheet 42) is applied to the main body 14 due to daily life, unexpected contact, or the like, the intermediate member 50 is elastically deformed, so that peeling can be avoided. For example, as shown in FIG. 4C, a case where an external force that separates the other end side of the case 20 from the body surface is applied will be described. In this case, the portion of the intermediate member 50 fixed to the main body 14 from the base 52 to each arm piece 58 is prevented from peeling from the main body 14. The portion of the intermediate member 50 fixed to the main-body-side attaching sheet 42 from the base 52 to the central piece 56 is prevented from peeling off from the main-body-side attaching sheet 42.

Therefore, even if the case 20 is separated from the body surface by an external force, the intermediate member 50 is elastically deformed as appropriate while being fixed to the main body 14 and the main-body-side attaching sheet 42. In the elastic deformation, while the coupling is continued at the base 52, the central piece 56 and the pair of arm pieces 58 are separated from each other to release an external force. At the time of elastic deformation of the elastic piece 54, the arc-shaped edge portion Ce of the base 52 where the central piece 56 and the pair of arm pieces 58 are coupled can suppress breakage of the intermediate member 50 even when an external force that tears the central piece 56 and the pair of arm pieces 58 is applied.

As described above, even in a case where the main body 14 is indwelled for a long period of time, the drug administration device 12 can greatly reduce peeling of the main-body-side attaching sheet 42 from the body surface. In particular, the rectangular main body 14 is easily peeled off along the longitudinal direction in the indwelling state, but since the elastic piece 54 is provided on the other end side in the longitudinal direction, the range in which the main body 14 and the main-body-side attaching sheet 42 are attached together is narrowed, and peeling of the main body 14 can be suppressed.

The drug administration device 12 having the indwelling structure 40 was attached to the abdomen of the examiner for 30 hours, and then an evaluation test of observing the attaching state was performed. As a result of the evaluation test, in the indwelling structure 40, about 5% of the entire area of the main-body-side attaching sheet 42 was peeled off, but it was confirmed that the main body 14 itself was not peeled off and the indwelling state could be preferably maintained.

The present invention is not limited to the above embodiments, and various modifications can be made. Hereinafter, some modifications of the indwelling device 10 according to the present invention will be described. Note that, in the description below, the same reference numerals are given to elements having the same configurations or the same functions as those of the above embodiment, and a detailed description thereof will be omitted.

### [First modification]

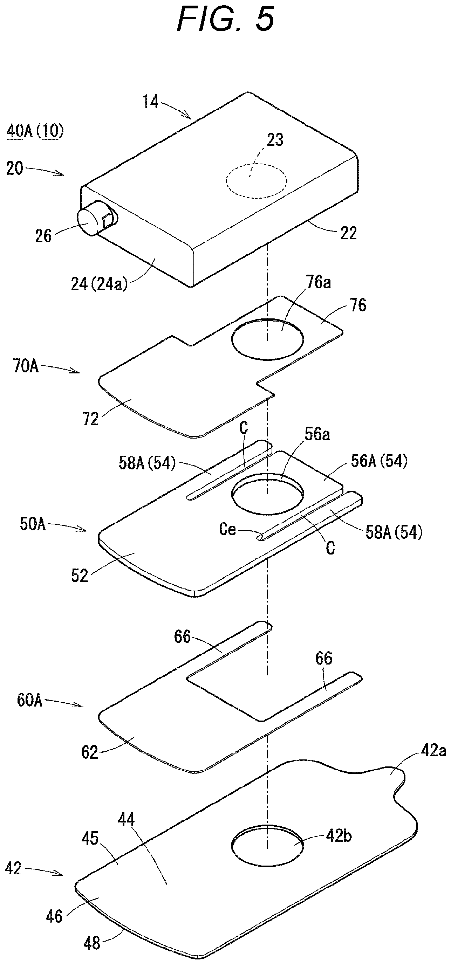

An indwelling structure 40A (intermediate member 50A) of the indwelling device 10 according to a first modification shown in FIG. 5 is different from the above-mentioned indwelling structure 40 in that it has a central piece 56A fixed to the main body 14, and has a pair of arm pieces 58A fixed to the main-body-side attaching sheet 42. In this case, the lower adhesive body 60A has a lower base attaching region 62 and a lower elastic piece attaching region 66 continuous with the lower base attaching region 62 and facing the pair of arm pieces 58A. Conversely, the upper adhesive body 70A has an upper base attaching region 72 and an elastic piece attaching region 76 continuous with the upper base attaching region 72 and facing the central piece 56A. In this case, the elastic piece attaching region 76 has the adhesive exposure hole 76a.

Even with such a configuration, in the indwelling structure 40A, while the pair of arm pieces 58A follows the body surface in the indwelling state of the main body 14, the central piece 56A follows the main body 14. Then, the central piece 56A and the pair of arm pieces 58A are relatively elastically deformed, so that it is possible to resist an external force.

### [Second modification]

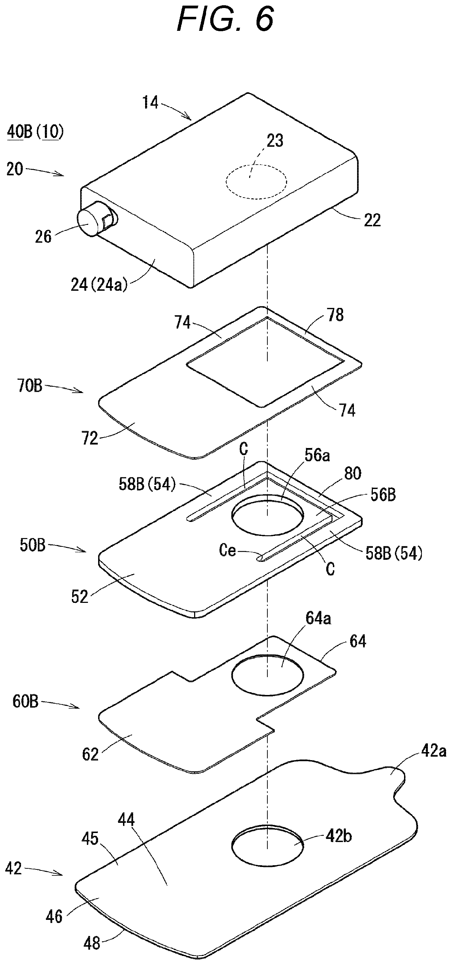

An indwelling structure 40B (intermediate member 50B) of the indwelling device 10 according to a second modification shown in FIG. 6 is different from the indwelling structures 40, 40A in that a central piece 56B extends short, and extending ends (the other ends) of a pair of arm pieces 58B are coupled to each other by a bridge portion 80. A lower adhesive body 60B is formed in the same shape as the indwelling structure 40 described above, while an upper adhesive body 70B has a bridge portion attaching region 78 which is continuous with the pair of upper elastic piece attaching regions 74 and faces the bridge portion 80.

As a result, in the indwelling structure 40B, the pair of arm pieces 58B and the bridge portion 80 can be more firmly fixed to the main body 14 (case 20). Therefore, the possibility that the intermediate member 50B is peeled off from the main body 14 due to the external force is further reduced while obtaining the same effect as the above-described indwelling structure 40. In other words, the shape of the portion (elastic piece 54) of the indwelling device 10 extending from the base 52 is not particularly limited, and various shapes may be adopted in consideration of the attaching range of the main body 14 or the main-body-side attaching sheet 42.

### [Third modification]

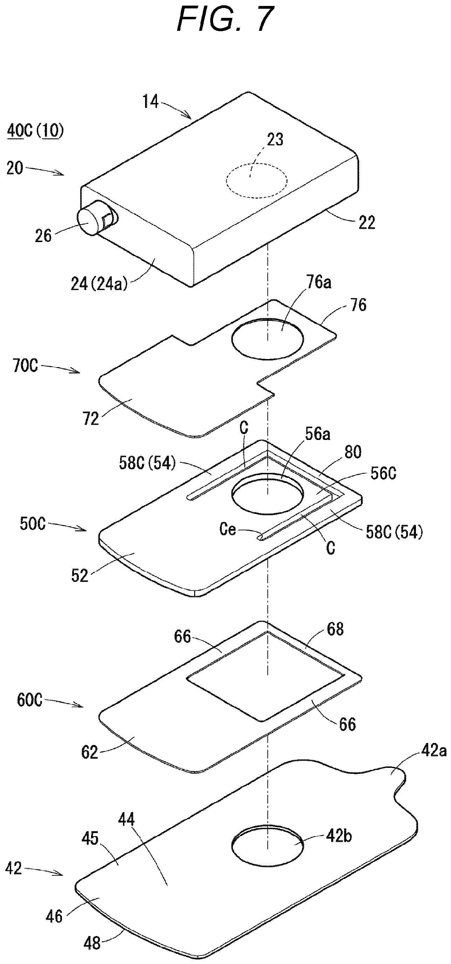

An indwelling structure 40C (intermediate member 50C) of the indwelling device 10 according to a third modification shown in FIG. 7 is different from the indwelling structures 40, 40A, 40B in that an intermediate member 50B having the same shape is applied, a central piece 56C is fixed to the main body 14, and a pair of arm pieces 58C and the bridge portion 80 are fixed to the main-body-side attaching sheet 42 via a lower adhesive body 60C. In this case, the central piece 56C is fixed to the main body 14 via an upper adhesive body 70C, and the pair of arm pieces 58C and the bridge portion 80 are fixed to the main-body-side attaching sheet 42 via a lower adhesive body 60C having the bridge portion attaching region 68 continuous with the pair of lower elastic piece attaching regions 66. Even with this configuration, the possibility that the intermediate member 50C is peeled off from the main-body-side attaching sheet 42 by an external force is further reduced while obtaining the same effect as the above-described indwelling structure 40.

### [Fourth modification]

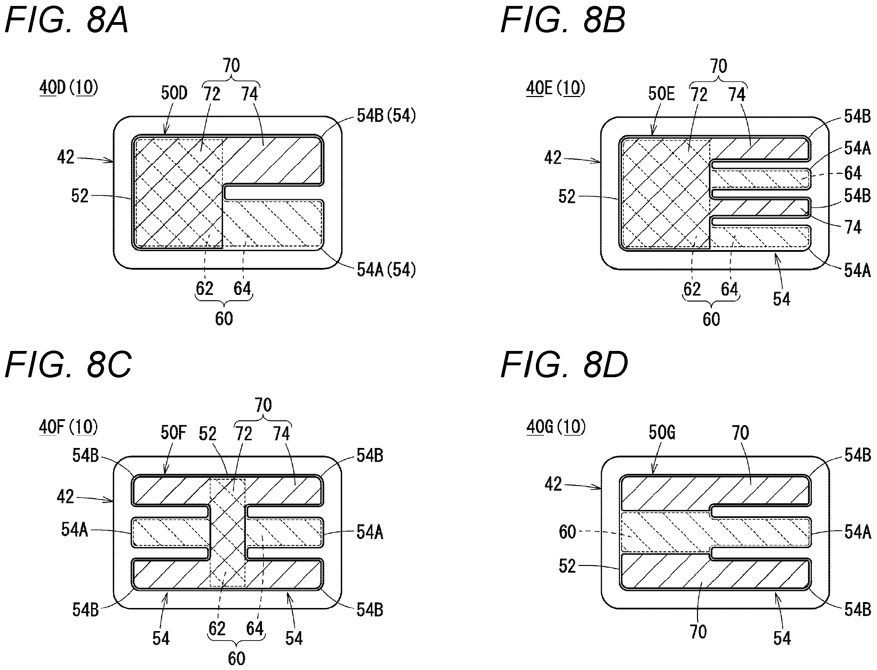

An indwelling structure 40D (intermediate member 50D) of the indwelling device 10 according to a fourth modification shown in FIG. 8A has two elastic pieces 54 extending in parallel and in the same direction from the base 52. One elastic piece 54A constitutes a first extending portion fixed to the main-body-side attaching sheet 42, and the other elastic piece 54B constitutes a second extending portion fixed to the main body 14. The indwelling device 10 can obtain the same effect as that of the indwelling structure 40 even if the indwelling structure 40D is configured as described above.

### [Fifth modification]

An indwelling structure 40E (intermediate member 50E) of the indwelling device 10 according to a fifth modification shown in FIG. 8B has four elastic pieces 54 extending in parallel and in the same direction from the base 52. In this case, the plurality of elastic pieces 54 may be alternately provided with a first extending portion (elastic piece 54A) fixed to the main-body-side attaching sheet 42 and a second extending portion (elastic piece 54B) fixed to the main body 14. In short, the number of the elastic pieces 54 provided in the intermediate members 50 and 50A to 50E can be arbitrarily designed.

### [Sixth modification]

An indwelling structure 40F (intermediate member 50F) of the indwelling device 10 according to a sixth modification shown in FIG. 8C has the base 52 at a central portion in the longitudinal direction. The indwelling structure 40F has a plurality of elastic pieces 54 extending in one end direction from the base 52 and a plurality of elastic pieces 54 extending in the other end direction from the base 52. Each of the plurality of elastic pieces 54 on one end side and the other end side has a first extending portion (elastic piece 54A) fixed to the main-body-side attaching sheet 42 and a second extending portion (elastic piece 54B) fixed to the main body 14. Even with such a configuration, the indwelling device 10 can be well indwelled on the curved body surface. Note that the plurality of elastic pieces 54 are not limited to extend in the longitudinal direction of the intermediate members 50, 50A to 50F, and may extend in the lateral direction.

### [Seventh modification]

An indwelling structure 40G (intermediate member 50G) of the indwelling device 10 according to a seventh modification shown in FIG. 8D has the base 52 and the plurality of elastic pieces 54, and has a first extending portion in which the base 52 is fixed to the main-body-side attaching sheet 42 and a second extending portion fixed to the main body 14. As described above, the intermediate member 50G can reinforce fixing between the main body 14 and the main-body-side attaching sheet 42 even if the lower adhesive body 60 and the upper adhesive body 70 do not have a portion overlapping in the vertical direction.

Technical ideas and effects that can be grasped from the above embodiments will be described below.

An indwelling device 10 according to one aspect of the present invention includes a main body 14, a sheet-like attaching member (main-body-side attaching sheet 42) for indwelling the main body on a body surface of a living body, and plate-like intermediate members 50, 50A to 50G that are configured to be elastically deformable and are stacked between the main body 14 and the attaching member, in which the intermediate members 50, 50A to 50G include a base 52 and a first extending portion (central piece 56, arm piece 58A) and a second extending portion (central piece 56A, arm piece 58) extending in parallel from the base 52, a first fixing portion (lower adhesive bodies 60, 60A to 60C) fixed to the attaching member from the base 52 to the first extending portion is provided on a surface of the intermediate members 50, 50A to 50G on the attaching member side, a second fixing portion (upper adhesive bodies 70, 70A to 70C) fixed to the main body 14 from the base 52 to the second extending portion is provided on a surface of the intermediate members 50, 50A to 50G on the main body 14 side, the first extending portion is not fixed to the main body 14, and the second extending portion is not fixed to the attaching member.

According to the above, the indwelling device 10 can stably indwell the main body 14 on the body surface of the living body by the simple configuration in which the intermediate members 50, 50A to 50G are provided between the main body 14 and the attaching member (main-body-side attaching sheet 42). That is, in the indwelling device 10 in a state of being indwelled on the body surface, the first extending portion (central piece 56, arm piece 58A) fixed to the attaching member follows the body surface without following the main body 14, and the second extending portion (central piece 56A, arm piece 58) fixed to the main body 14 can be separated from the body surface. Therefore, even if an external force is applied to the main body 14, the intermediate members 50, 50A to 50G elastically deform to absorb the external force while maintaining the fixing between the first fixing portion (lower adhesive bodies 60, 60A to 60C) and the attaching member and the fixing between the second fixing portion (upper adhesive body 70, 70A to 70C) and the main body 14. Therefore, even in a case where the indwelling device 10 is indwelled on the body surface for a long period of time, it is possible to maintain the indwelling while suppressing the attaching state of the attaching member from becoming defective.

The first fixing portion (lower adhesive bodies 60, 60A to 60C) is provided on the entire surface of the base 52 on the attaching member (main-body-side attaching sheet 42) side, and the second fixing portion (upper adhesive bodies 70, 70A to 70C) is provided on the entire surface of the base 52 on the main body 14 side. As a result, the intermediate members 50, 50A to 50G can firmly indwell the main body 14 at the base 52.

The intermediate members 50, 50A to 50E are formed in a rectangular shape coinciding with the fixing target place of the main body 14 in plan view, have the base 52 on one end side in the longitudinal direction, and have the first extending portion (central piece 56, arm piece 58A) and the second extending portion (central piece 56A, arm piece 58) on the other end side in the longitudinal direction. The rectangular main body 14 is easily peeled off along the longitudinal direction in the indwelling state, but since the indwelling device 10 has the first extending portion and the second extending portion on the other end side in the longitudinal direction as described above, it is possible to suppress peeling of the main body 14 from the longitudinal direction.

One of the first extending portion (central piece 56, arm piece 58A) and the second extending portion (central piece 56A, arm piece 58) is a central piece 56, 56A protruding from a central portion of the base 52 in the width direction, and the other of the first extending portion and the second extending portion is a pair of arm pieces 58, 58A protruding from both sides of the base 52 in the width direction. As a result, the indwelling device 10 can further stabilize the fixing of the intermediate members 50, 50A to 50C to the attaching member (main-body-side attaching sheet 42) and the main body 14.

The pair of arm pieces 58, 58A includes a bridge portion 80 that bridges the protruding ends of the arm pieces. As a result, the intermediate members 50B, 50C can more firmly fix the pair of arm pieces 58, 58A and the bridge portion 80 to the attaching member (main-body-side attaching sheet 42) or the main body 14.

A clearance C is provided between the first extending portion (central piece 56, arm piece 58A) and the second extending portion (central piece 56A, arm piece 58) adjacent to each other, and the clearance C has an arc-shaped edge portion Ce at a portion where the first extending portion and the second extending portion are coupled to the base 52. As a result, an external force that tears the first extending portion and the second extending portion is applied to the intermediate members 50, 50A to 50G, so that it is possible to suppress the damage at the edge portion Ce and maintain the indwelling state preferably.

The main body 14 has the operation unit 23 for operating the main body 14 on a surface facing the intermediate members 50, 50A to 50C, the intermediate members 50, 50A to 50C have an exposure portion (intermediate member side exposure hole 56a) for exposing the operation unit 23 at a position facing the operation unit 23, and the exposure portion is provided in either the first extending portion (central piece 56) or the second extending portion (central piece 56A). As a result, the indwelling device 10 can reliably operate the operation unit 23 even in the configuration including the intermediate members 50, 50A to 50C. In particular, even after the user forgets to operate the operation unit 23 and indwells the main body 14 on the body surface, if the operation unit 23 is at the position of the first extending portion or the second extending portion, it is possible to operate the operation unit 23 while exposing the operation unit 23 without peeling the attaching member (the main-body-side attaching sheet 42) from the body surface.

The indwelling device 10 is a drug administration device 12 that administers a drug to a living body, and the base 52 is disposed at a position near an outflow portion of the drug of the main body 14. As a result, the drug administration device 12 can firmly hold the drug outflow portion of the main body 14 at the base 52 of the intermediate members 50, 50A to 50E, 50G.

## Claims

1. An indwelling device (10) comprising:
a main body (14);
a sheet-like attaching member (42) for indwelling the main body (14) on a body surface of a living body; and
a plate-like intermediate member (50, 50A to 50G) that is configured to be elastically deformable and is stacked between the main body (14) and the attaching member (42),
wherein the intermediate member (50, 50A to 50G) includes a base (52), and a first extending portion (56, 58A) and a second extending portion (56A, 58) that extend in parallel from the base (52),
a first fixing portion (60, 60A to 60C) fixed to the attaching member (42) from the base (52) to the first extending portion (56, 58A) is provided on a surface of the intermediate member (50, 50A to 50G) on a side of the attaching member (42),
a second fixing portion (70, 70A to 70C) fixed to the main body (14) from the base (52) to the second extending portion (56A, 58) is provided on a surface of the intermediate member (50, 50A to 50G) on a side of the main body (14),
the first extending portion (56, 58A) is not fixed to the main body (14), and
the second extending portion (56A, 58) is not fixed to the attaching member (42), wherein
one of the first extending portion (56, 58A) and the second extending portion (56A, 58) is a central piece (56, 56A-C) protruding from a central portion in a width direction of the base (52), and
another one of the first extending portion (56, 58A) and the second extending portion (56A, 58) is a pair of arm pieces (58, 58A-C) protruding from both sides in the width direction of the base (52).

2. The indwelling device (10) according to claim 1,
wherein the first fixing portion (60, 60A to 60C) is provided on an entire surface of the base (52) on a side of the attaching member (42), and
the second fixing portion (70, 70A to 70C) is provided on an entire surface of the base (52) on a side of the main body (14).

3. The indwelling device (10) according to claim 1 or 2,
wherein the intermediate member (50, 50A to 50G) is formed in a rectangular shape coinciding with a fixing target place of the main body (14) in plan view, and
includes the base (52) on one end side in a longitudinal direction, and the first extending portion (56, 58A) and the second extending portion (56A, 58) on another end side in the longitudinal direction.

4. The indwelling device (10) according to claim 3,
wherein the pair of arm pieces (58, 58A-C) includes a bridge portion (80) that bridges protruding ends of the pair of arm pieces (58, 58A-C).

5. The indwelling device (10) according to any one of claims 1 to 4,
wherein a clearance (C) is provided between the first extending portion (56, 58A) and the second extending portion (56A, 58) adjacent to each other, and
the clearance (C) has an arc-shaped edge portion (Ce) at a portion where the first extending portion (56, 58A) and the second extending portion (56A, 58) are coupled to the base (52).

6. The indwelling device (10) according to any one of claims 1 to 5,
wherein the main body (14) includes an operation unit (23) for operating the main body (14) on a surface facing the intermediate member (50, 50A to 50G),
the intermediate member (50, 50A to 50G) includes an exposure portion (56a) for exposing the operation unit (23) at a position facing the operation unit (23), and
the exposure portion (56a) is provided in either the first extending portion (56, 58A) or the second extending portion (56A, 58).

7. The indwelling device (10) according to any one of claims 1 to 6,
wherein the indwelling device (10) is a drug administration device (12) that administers a drug to the living body, and
the base (52) is disposed at a position near an outflow portion of the drug of the main body (14).

## Patentansprüche

1. Verweilvorrichtung (10), umfassend:
einen Hauptkörper (14);
ein blattförmiges Befestigungselement (42) zum Verweilen des Hauptkörpers (14) an einer Körperoberfläche eines lebenden Körpers; und
ein plattenförmiges Zwischenelement (50, 50A bis 50G), das so konfiguriert ist, dass es elastisch verformbar ist und zwischen dem Hauptkörper (14) und dem Befestigungselement (42) gestapelt ist,
wobei das Zwischenelement (50, 50A bis 50G) eine Basis (52) und einen ersten sich erstreckenden Abschnitt (56, 58A) und einen zweiten sich erstreckenden Abschnitt (56A, 58) aufweist, die sich parallel von der Basis (52) erstrecken,
einen ersten Befestigungsabschnitt (60, 60A bis 60C), der an dem Befestigungselement (42) von der Basis (52) bis zu dem ersten sich erstreckenden Abschnitt (56, 58A) befestigt ist, der auf einer Oberfläche des Zwischenelements (50, 50A bis 50G) auf einer Seite des Befestigungselements (42) vorgesehen ist,
einen zweiten Befestigungsabschnitt (70, 70A bis 70C), der an dem Hauptkörper (14) von der Basis (52) bis zu dem zweiten sich erstreckenden Abschnitt (56A, 58) befestigt ist, der auf einer Oberfläche des Zwischenelements (50, 50A bis 50G) auf einer Seite des Hauptkörpers (14) vorgesehen ist,
der erste sich erstreckende Abschnitt (56, 58A) nicht an dem Hauptkörper (14) befestigt ist, und
der zweite sich erstreckende Abschnitt (56A, 58) nicht an dem Befestigungselement (42) befestigt ist,
wobei einer von dem ersten sich erstreckenden Abschnitt (56, 58A) und dem zweiten sich erstreckenden Abschnitt (56A, 58) ein Mittelteil (56, 56A-C) ist, das von einem Mittelabschnitt in einer Breitenrichtung der Basis (52) hervorsteht, und
ein anderer von dem ersten sich erstreckenden Abschnitt (56, 58A) und dem zweiten sich erstreckenden Abschnitt (56A, 58) ein Paar von Armstücken (58, 58A-C) ist, die von beiden Seiten in der Breitenrichtung der Basis (52) hervorstehen.

2. Verweilvorrichtung (10) nach Anspruch 1,
wobei der erste Befestigungsabschnitt (60, 60A bis 60C) auf einer gesamten Oberfläche der Basis (52) auf einer Seite des Befestigungselements (42) vorgesehen ist, und der zweite Befestigungsabschnitt (70, 70A bis 70C) auf einer gesamten Oberfläche der Basis (52) auf einer Seite des Hauptkörpers (14) vorgesehen ist.

3. Verweilvorrichtung (10) nach Anspruch 1 oder 2,
wobei das Zwischenelement (50, 50A bis 50G) in einer rechteckigen Form ausgebildet ist, die in der Draufsicht mit einem Zielort der Befestigung des Hauptkörpers (14) zusammenfällt, und
die Basis (52) an einer Endseite in einer Längsrichtung und den ersten sich erstreckenden Abschnitt (56, 58A) und den zweiten sich erstreckenden Abschnitt (56A, 58) an einer anderen Endseite in der Längsrichtung umfasst.

4. Verweilvorrichtung (10) nach Anspruch 3,
wobei das Paar von Armstücken (58, 58A-C) einen Brückenabschnitt (80) aufweist, der die hervorstehenden Enden des Paares von Armstücken (58, 58A-C) überbrückt.

5. Verweilvorrichtung (10) nach einem der Ansprüche 1 bis 4,
wobei ein Zwischenraum (C) zwischen dem ersten sich erstreckenden Abschnitt (56, 58A) und dem zweiten sich erstreckenden Abschnitt (56A, 58) angrenzend aneinander vorgesehen ist, und
der Zwischenraum (C) einen bogenförmigen Randabschnitt (Ce) an einem Abschnitt aufweist, an dem der erste sich erstreckende Abschnitt (56, 58A) und der zweite sich erstreckende Abschnitt (56A, 58) mit der Basis (52) verbunden sind.

6. Verweilvorrichtung (10) nach einem der Ansprüche 1 bis 5,
wobei der Hauptkörper (14) eine Betätigungseinheit (23) zum Betätigen des Hauptkörpers (14) auf einer dem Zwischenelement (50, 50A bis 50G) zugewandten Fläche aufweist,
das Zwischenelement (50, 50A bis 50G) einen Freilegungsabschnitt (56a) zum Freilegen der Betätigungseinheit (23) an einer der Betätigungseinheit (23) zugewandten Position aufweist, und
der Freilegungsabschnitt (56a) entweder in dem ersten sich erstreckenden Abschnitt (56, 58A) oder in dem zweiten sich erstreckenden Abschnitt (56A, 58) vorgesehen ist.

7. Verweilvorrichtung (10) nach einem der Ansprüche 1 bis 6,
wobei die Verweilvorrichtung (10) eine Vorrichtung (12) zur Verabreichung von Arzneimitteln ist, die dem lebenden Körper ein Arzneimittel verabreicht, und
die Basis (52) an einer Position in der Nähe eines Ausflussabschnitts des Arzneimittels von dem Hauptkörper (14) angeordnet ist.

## Revendications

1. Dispositif à demeure (10) comprenant :
un corps principal (14) ;
un élément d'attache de type feuille (42) pour la pose à demeure du corps principal (14) sur une surface corporelle d'un corps vivant ; et
un élément intermédiaire de type plaque (50, 50A à 50G) qui est configuré pour être élastiquement déformable et est empilé entre le corps principal (14) et l'élément d'attache (42),
dans lequel l'élément intermédiaire (50, 50A à 50G) comporte une base (52), et une première partie d'extension (56, 58A) et une deuxième partie d'extension (56A, 58) qui s'étendent parallèlement à partir de la base (52),
une première partie de fixation (60, 60A à 60C) fixée à l'élément d'attache (42) depuis la base (52) jusqu'à la première partie d'extension (56, 58A) est disposée sur une surface de l'élément intermédiaire (50, 50A à 50G) sur un côté de l'élément d'attache (42),
une deuxième partie de fixation (70, 70A à 70C) fixée au corps principal (14) depuis la base (52) jusqu'à la deuxième partie d'extension (56A, 58) est disposée sur une surface de l'élément intermédiaire (50, 50A à 50G) sur un côté du corps principal (14),
la première partie d'extension (56, 58A) n'est pas fixée au corps principal (14), et
la deuxième partie d'extension (56A, 58) n'est pas fixée à l'élément d'attache (42),
dans lequel l'une de la première partie d'extension (56, 58A) et de la deuxième partie d'extension (56A, 58) est une pièce centrale (56, 56A-C) faisant saillie à partir d'une partie centrale dans une direction de largeur de la base (52), et
l'autre de la première partie d'extension (56, 58A) et de la deuxième partie d'extension (56A, 58) est une paire de pièces en forme de bras (58, 58A-C) faisant saillie à partir des deux côtés dans la direction de largeur de la base (52).

2. Dispositif à demeure (10) selon la revendication 1,
dans lequel la première partie de fixation (60, 60A à 60C) est disposée sur une surface entière de la base (52) sur un côté de l'élément d'attache (42), et
la deuxième partie de fixation (70, 70A à 70C) est disposée sur une surface entière de la base (52) sur un côté du corps principal (14).

3. Dispositif à demeure (10) selon la revendication 1 ou 2, dans lequel l'élément intermédiaire (50, 50A à 50G) se présente sous une forme rectangulaire coïncidant avec un emplacement cible de fixation du corps principal (14) dans une vue en plan, et
comporte la base (52) sur un premier côté d'extrémité dans une direction longitudinale, et la première partie d'extension (56, 58A) et la deuxième partie d'extension (56A, 58) sur un deuxième côté d'extrémité dans la direction longitudinale.

4. Dispositif à demeure (10) selon la revendication 3,
dans lequel la paire de pièces en forme de bras (58, 58A-C) comporte une partie pont (80) qui couvre des extrémités en saillie de la paire de pièces en forme de bras (58, 58A-C).

5. Dispositif à demeure (10) selon l'une quelconque des revendications 1 à 4,
dans lequel un jeu (C) est prévu entre la première partie d'extension (56, 58A) et la deuxième partie d'extension (56A, 58) adjacentes l'une à l'autre, et
le jeu (C) a une partie de bord en forme d'arc (Ce) au niveau d'une partie où la première partie d'extension (56, 58A) et la deuxième partie d'extension (56A, 58) sont accouplées à la base (52).

6. Dispositif à demeure (10) selon l'une quelconque des revendications 1 à 5,
dans lequel le corps principal (14) comporte une unité d'actionnement (23) pour actionner le corps principal (14) sur une surface faisant face à l'élément intermédiaire (50, 50A à 50G),
l'élément intermédiaire (50, 50A à 50G) comporte une partie d'exposition (56a) pour exposer l'unité d'actionnement (23) à une position faisant face à l'unité d'actionnement (23), et
la partie d'exposition (56a) est disposée soit dans la première partie d'extension (56, 58A) soit dans la deuxième partie d'extension (56A, 58).

7. Dispositif à demeure (10) selon l'une quelconque des revendications 1 à 6,
dans lequel le dispositif à demeure (10) est un dispositif d'administration de médicament (12) qui administre un médicament au corps vivant, et
la base (52) est disposée à une position à proximité d'une partie de décharge du médicament du corps principal (14).
